## Europäisches Patentamt
## European Patent Office
## Office européen des brevets

(19)

(11) Veröffentlichungsnummer : **0 133 272**
**B1**

(12) # EUROPÄISCHE PATENTSCHRIFT

(45) Veröffentlichungstag der Patentschrift :
**11.10.89**

(51) Int. Cl.⁴ : **G 01 N 33/543**

(21) Anmeldenummer : **84108708.3**

(22) Anmeldetag : **24.07.84**

(54) **Verfahren zur Bestimmung eines Partners einer Immunreaktion sowie Reagens zur Durchführung dieses Verfahrens.**

(30) Priorität : **30.07.83 DE 3327642**

(43) Veröffentlichungstag der Anmeldung :
**20.02.85 Patentblatt 85/08**

(45) Bekanntmachung des Hinweises auf die Patenterteilung : **11.10.89 Patentblatt 89/41**

(84) Benannte Vertragsstaaten :
**AT BE CH DE FR GB IT LI LU NL SE**

(56) Entgegenhaltungen :
EP--A-- 0 132 948
US--A-- 4 060 597
US--A-- 4 200 436
US--A-- 4 241 175
US--A-- 4 273 756
US--A-- 4 311 788
US--A-- 4 313 927
US--A-- 4 357 310
US--A-- 4 454 232

(73) Patentinhaber : **BOEHRINGER MANNHEIM GMBH**
**Sandhofer Strasse 116**
**D-6800 Mannheim 31 (DE)**

(72) Erfinder : **Baier, Manfred, Dr. rer. nat.**
**Tiefentalweg 10**
**D-8124 Seeshaupt (DE)**

EP 0 133 272 B1

**Beschreibung**

Die Erfindung betrifft ein Verfahren zur Bestimmung eines Partners einer Immunreaktion, bei der einer der Reaktionspartner an einen festen Träger adsorbiert ist, in einer Serum- oder Plasma-Probe, wobei dem Inkubationsmilieu der Immunreaktion zwischen dem löslichen Partner bzw. den löslichen Partnern und dem an den festen Träger adsorbierten Partner ein Detergens zugegeben wird.

Es ist seit mehreren Jahren bekannt, daß immunologische Bestimmungen im Serum und im Plasma zu unterschiedlichen Ergebnissen führen. Es werden hierfür inhibitorische Faktoren verantwortlich gemacht, die sich in Blutplasma befinden und die Aktivität des an einen festen Träger adsorbierten Bindungspartners beeinflussen.

In Clin. Chem. 24 (1978) S. 137-139 werden beispielsweise für zahlreiche radioimmunologische Bestimmungen die Unterschiede zwischen Serum- und Plasma-Proben aufgezeigt. Daß auch für einen Enzym-Immunoassay unterschiedliche Werte für Messungen im Serum und im Plasma gefunden werden, wird beispielsweise in Ärztl. Lab. 27 (1981) S. 69-73 beschrieben. Es wird in dieser Arbeit auch die Schlußfolgerung gezogen, daß derartige Inhaltsstoffe des Plasmas, vor allem Fibrinogen, bei allen Enzym-Immunoassays, bei denen das Prinzip der heterogenen Verfahrensweise zur Anwendung kommt, zu Störungen der Immunreaktion zwischen den adsorbierten und dem löslichen Partner bzw. den löslichen Partnern führen.

Es läßt sich allerdings in der Praxis nicht generell vermeiden, auch Plasma als Probenmilieu einzusetzen. Ferner ist es zuweilen bei einer zu messenden Probe überhaupt nicht bekannt, ob sie auf der Basis eines Serums oder Plasmas zubereitet worden ist.

Es besteht somit ein Bedarf nach einem Verfahren zur Bestimmung eines Partners einer Immunreaktion, bei dem ein Partner in an einen festen Träger adsorbierter Form vorliegt, das unabhängig vom Probenmaterial, Serum oder Plasma, zuverlässige und übereinstimmende Werte liefert. In Ärztl. Lab. 27 (1981) S. 69-73 wird vorgeschlagen, zur Beseitigung der genannten Störung die Probe für 10 Minuten auf 56 °C zu erhitzen und anschließend die denaturierten Störsubstanzen abzuzentrifugieren. Dieser Hitzeschritt mit anschließender Zentrifugation bedeutet jedoch bei der praktischen Durchführung der Bestimmungsverfahren zusätzliche Arbeitsschritte. Darüber hinaus ist damit zu rechnen, daß durch die Hitzebehandlung andersartige Störungen und Fehlerquellen hervorgerufen werden.

Aufgabe der Erfindung war es, ein Verfahren bereitzustellen, das sowohl in Serum- als auch in Plasma-Proben vergleichbare Werte liefert und darüber hinaus auf einfache Weise und ohne zusätzliche Verfahrensschritte durchführbar ist.

Die Aufgabe wird gelöst durch ein Verfahren zur vergleichbaren Bestimmung eines Partners einer Immunreaktion in Serum- und Plasmaproben, bei der einer der Reaktionspartner an einen festen Träger adsorbiert ist, dadurch gekennzeichnet, daß dem Inkubationsmilieu der Immunreaktion zwischen dem löslichen Partner bzw. den löslichen Partnern und dem an den festen Träger adsorbierten Bindungspartner ein Detergens zugegeben wird, so daß die Detergens-Konzentration 0,0001 bis 0,01 % (Gew./Vol.) beträgt.

Ein weiterer Gegenstand der Erfindung ist ein Reagens zur Durchführung einer Immunreaktion zwischen einem an einen festen Träger adsorbierten Reaktionspartner und einem löslichen Partner bzw. löslichen Partnern in einer Serum- oder Plasma-Probe, das zusätzlich zu den für die Immunreaktion üblicherweise erforderlichen Bestandteile ein Detergens in einer Konzentration von 0,0001-0,01 (Gew./Vol.) enthält. Die Detergens-Konzentration im Reagens beträgt besonders bevorzugt 0,001-0,005 % (Gew./Vol.). Das erfindungsgemäße Reagens kann als Pulver, Lyophilisat oder als Lösung vorliegen.

Als Detergentien können nicht-ionische und anionische Detergentien eingesetzt werden. Beispiele für nicht-ionische Detergentien sind Epoxy-Fettsäure-Ester, beispielsweise Tween 20 (Polyoxyethylen-sorbitan-monolaurat), Tween 80 (Polyoxy-ethylen-sorbitan-monooleat), Triton (Alkyl-polyether-alkohol-Gemische), Brij 35 (Polyoxyethylenlauryl-ether), Non-Idet P-40 (Polyoxy-ethylen-octylphenol-ether), Lubrol PX (Polyethylenoxid-alkylether-Addukt), Berol EMU 043 (C-16, C-18-Fettalkohol mit 10 Oxyethyleneinheiten) u. ä. Als Beispiel für ein anionisches Detergens sei Natriumdodecyl-sulfat (SDS) angeführt.

Vorzugsweise werden die Detergentien der Inkubationslösung in einer Konzentration von 0,001-0,005 % (Gew./Vol.) zugesetzt.

Es war bekannt, durch Zugabe von Detergentien unspezifische Bindungen bei einer Immunreaktion zu unterdrücken bzw. zu beseitigen. Wird bei einer Immunreaktion ein Bindungspartner in an einen festen Träger adsorbierter Form eingesetzt, so wurde ein Zusatz von Detergentien bisher vermieden, da dadurch die adsorptive Bindung des Bindungspartners an den Träger geschwächt bzw. völlig aufgehoben wird.

So beschreibt US-A-4,241,175, daß zur Adsorption von Antigen an Trägermaterialien nicht-ionische Detergentien eingesetzt werden können. Beispielhaft offenbart sind Konzentrationen von 0,1 und 1 Vol.-%. Die eigentliche Immunreaktion wird nicht in Anwesenheit von Detergens durchgeführt, sondern nur die Fixierung eines der Reaktanden auf einem festen Träger zur Herstellung eines der für einen Immunoassay notwendigen Ausgangsreagentien.

US-A-4,273,756 beschreibt die Verwendung von 0,2 bzw. 1 % Tween-20 in Immunoassays für klassenspezifische Antikörper in Serum. Der Patentschrift kann nicht entnommen werden, ob unter den beispielhaft offenbarten Bedingungen mit Plasma die gleichen Ergebnisse erzielt werden.

Ebenso werden auch in US-A-4,313,927 beispielhaft Detergenskonzentrationen von 0,05 % in Radioimmunoassays beschrieben.

US-A-4,357,310 betrifft die Erhöhung der Lagerstabilität eines radioaktiv markierten Proteinhormons, das für Radioimmunoassays verwendet werden soll durch Detergenszusatz. Die beispielhaften Detergenskonzentrationen liegen bei 0,5 % (Gew./Vol.). Zu dem Problem der unterschiedlichen Resultate bei Immunoassays in Serum und Plasma wird in dieser Patentschrift nicht Stellung genommen.

In US-A-4,060,597 wird die Stabilisierung von Latexreagentien für serologische Untersuchungen beschrieben, die auf Latexagglutination basieren. Speziell betrifft die Schrift die Stabilisierung von Polystyrol-Latexpartikeln, die vor der Beladung mit serologisch aktiven Substanzen mit einem nichtionischen Detergens behandelt wurden. Demgegenüber soll die vorliegende Erfindung ein Verfahren und Reagens für Immunoassays schaffen, das bei der Anwendung auf Serum- und Plasma-Proben gleichermaßen zuverlässige und übereinstimmende Resultate ergibt. Hierzu kann US-A-4,060,597 nichts entnommen werden.

Auch US-A-4,311,788 betrifft eine nephelometrische Methode. Es wird dort beschrieben, wie trübungsverursachende Stoffe, die die nephelometrische Messung stören, durch Zusatz von Detergentien unwirksam gemacht werden. Als ergänzende Maßnahme wird ein enzymatischer Abbau solcher trübungsverursachender Stoffe empfohlen. Die Literaturstelle gibt keinerlei Hinweis darauf, wie übereinstimmende Resultate zwischen Serum- und Plasma-Proben erzielt werden können.

Es war deshalb überraschend, daß der erfindungsgemäße Zusatz von Detergentien in der genannten geringen Konzentration ausreicht, die Wirkung der inhibitorischen Faktoren des Blutplasmas vollständig auszuschalten, ohne die adsorptive Bindung des Bindungspartners an den festen Träger zu beeinträchtigen.

Unter einer Immunreaktion im Sinne der Erfindung sind alle Reaktionen zwischen immunologischen Rezeptoren und Akzeptoren zu verstehen. Erfindungswesentlich ist nur, daß ein Reaktionspartner bei der Reaktion oder aber auch nur bei einem Teilschritt der Gesamtreaktion in an einen festen Träger adsorbierter Form eingesetzt wird.

Beispielhaft für die zahlreichen bekannten Varianten solcher immunologischer Bestimmungsverfahren sei der kompetitive Assay erwähnt, bei dem zur Bestimmung eines Akzeptors in einer Probe eine bekannte Menge des markierten Akzeptors zugesetzt wird und der Akzeptor und der markierte Akzeptor um im Unterschuß vorhandene Rezeptorbindungsstellen konkurrieren, die sich auf einem festen Träger befinden. Ein weiteres breites Gebiet sind die Sandwich-Teste, bei denen ein mindestens bivalenter Akzeptor mit einem markierten Rezeptor und einem an einen festen Träger adsorbierten Rezeptor umgesetzt wird. Diese Umsetzung kann auf verschiedene Weise erfolgen :

a) Der Akzeptor wird gleichzeitig mit dem markierten und dem unlöslichen Rezeptor umgesetzt.

b) Im ersten Schritt erfolgt die Umsetzung zwischen Akzeptor und dem unlöslichen Rezeptor. Der entstehende Komplex wird in einem zweiten Schritt mit dem markierten Rezeptor inkubiert.

c) Im ersten Schritt erfolgt die Umsetzung zwischen dem Akzeptor und dem markierten Rezeptor. Der entstandene lösliche Komplex wird anschließend mit dem unlöslichen Rezeptor umgesetzt.

Der erfindungsgemäße Zusatz des Detergens erfolgt jeweils in dem Schritt, in dem der an einen festen Träger adsorbierte Rezeptor an der Reaktion teilnimmt.

Unter einem Akzeptor ist insbesondere ein Antigen oder Hapten zu verstehen. Als Rezeptoren kommen vollständige Antikörper oder auch Antikörper-Fragment infrage. Die Markierung kann in bekannter Weise durch ein Enzym, ein Radioisotop, eine fluoreszierende Verbindung usw. erfolgen. Als fester Träger eignen sich Platten, Kugeln, Röhrchen, Streifen oder Stäbchen aus Glas oder Kunststoffen.

Die folgenden Beispiele erläutern die Erfindung :

Beispiel 1

Bestimmung von Digoxin mit Hilfe eines kompetitiven ELISA-Tests

Schaf-anti-Digoxin wird je nach Titer in geeigneter Weise mit Phosphatpuffer, 10 mM, pH 7,5, verdünnt, in Reagensröhrchen aus Polystyrol gefüllt und über Nacht inkubiert. In die Röhrchen werden ferner 1000 µl Konjugatpuffer, der Digoxin-Peroxidase (20 mU/ml) in Phosphatpuffer, 40 mM, pH 6,8, Rinderserumalbumin 0,25 % sowie einmal kein und einmal 0,003 % Tween® 20 enthält, sowie 100 µl digoxinhaltiges Serum bzw. Plasma bzw. Standardprobe pipettiert. Das Gemisch wird eine Stunde bei Raumtemperatur inkubiert. Danach werden die Röhrchen ausgesaugt und mit Leitungswasser gewaschen.

Die kovalente Bindung (Kopplung) von Peroxidase an Antikörper erfolgt nach der Methode von M. B. Wilson, Paul K. Nakane, beschrieben in « Recent Developments in the Perjodate Methode of Conjugating Horse Radish Peroxidase (HRPO) to Antibodies ». 1978 Elsevier/North-Holland Biomedical Press Seite 215-224 in « Immunfluorescence and Related Staining Techniques ».

Anschließend wird die Peroxidase-Aktivität an der Oberfläche der Polystyrolröhrchen in bekannter Weise bestimmt. Hierzu werden 1000 µl Substratlösung, die ABTS und Wasserstoffperoxid in 100 mM Phosphat/Citrat-Puffer, pH 4,4, enthält, in die Röhrchen pipettiert und eine Stunde bei Raumtemperatur inkubiert. Anschließend werden die Extinktionen am Photometer vermessen. Durch Messen von Standardproben mit bekannter Digoxinkonzentration wird eine Eichkurve erstellt. Aus der Extinktion der Proben mit unbekannter Digoxinkonzentration wird mit Hilfe dieser Eichkurve die Digoxinkonzentration in der unbekannten Probe bestimmt.

ABTS = 2,2'-Azino-di[3-ethyl-benzthiazolin-sulfonat(6)].

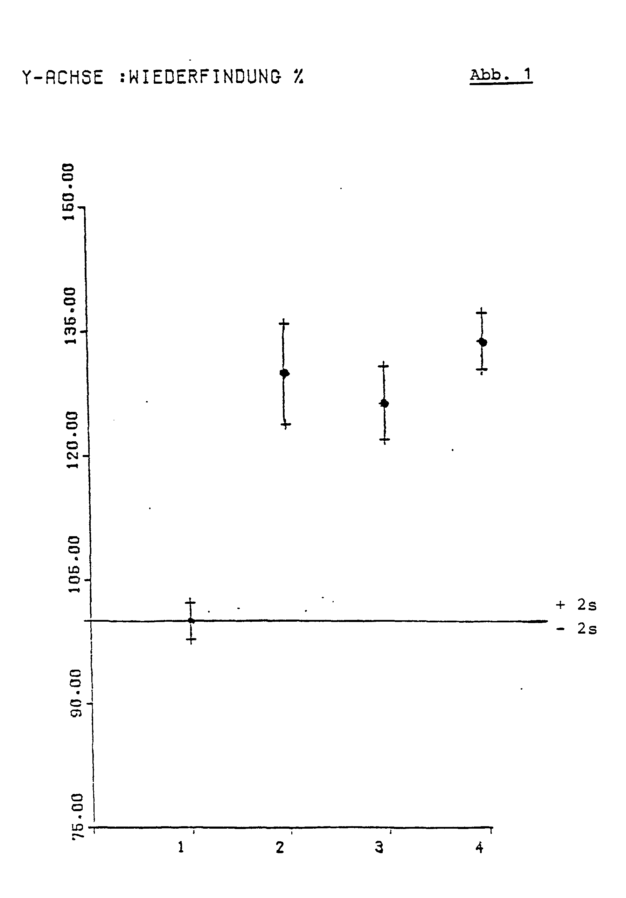

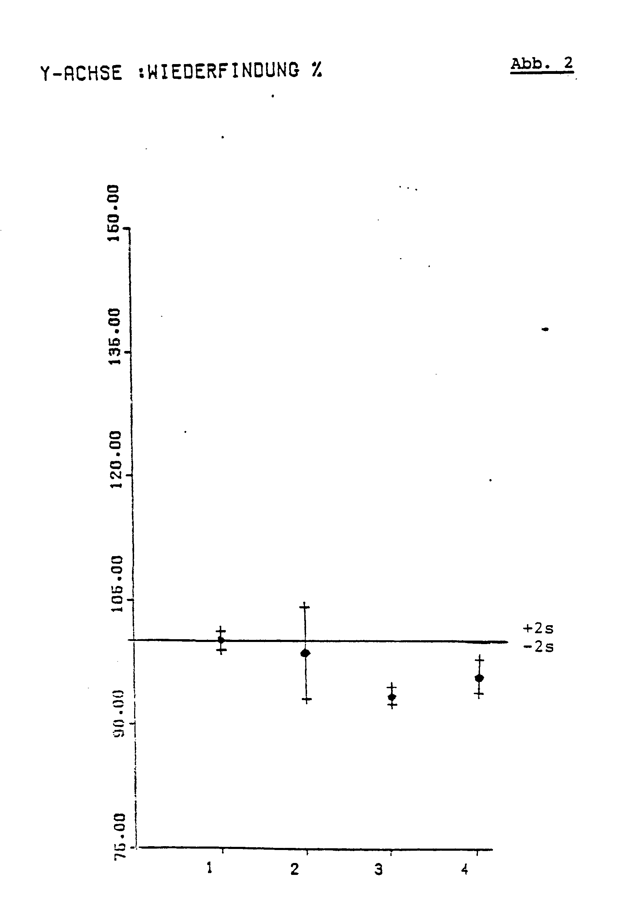

In Abbildung 1 ist die Digoxin-Wiederfindungsrate in % für verschiedene Probenmedien dargestellt, wenn die oben beschriebene Testdurchführung in Abwesenheit eines Detergens durchgeführt wird. 1 zeigt den 100 %-Wert für klares Humanserum, das 1,0 ng/ml Digoxin enthält. Die entsprechenden Werte für verschiedene Plasmaproben : 2 = Oxalat-Plasma, 3 = Citrat-Plasma und 4 = Heparin-Plasma sind in Abbildung 1 ebenfalls angegeben. Die Werte zeigen deutlich, daß in Abwesenheit eines Detergens eine wesentlich erhöhte Digoxin-Konzentration vorgetäuscht wird. In Abbildung 2 sind die korrespondierenden Werte für Messungen angegeben, die in völlig analoger Weise jedoch jetzt mit Zusatz von 0,003 % Tween® 20, wie vorstehend angegeben, durchgeführt worden sind. Die in Abbildung 2 aufgeführten Werte zeigen, daß in letzterem Falle nur geringfügige Abweichungen zu beobachten sind.

Beispiel 2

Bestimmung von AFP (alpha-1-Fötoprotein) mit Hilfe eines Sandwich-ELISA-Tests

Wie in Beispiel 1 beschrieben, werden Kunststoffröhrchen mit Schaf-anti-AFP adsorptiv beschichtet. Die Röhrchen werden getrocknet und anschließend wie folgt verwendet :
1 000 µl eines Inkubationspuffers, der aus 100 mM Phosphat-Puffer, pH 6,8 und 1 % Rinderserumalbumin besteht, und 200 µl Probe bzw. Standard werden in die Röhrchen gegeben und eine Stunde bei Raumtemperatur inkubiert. Anschließend wird ausgesaugt, mit Leitungswasser einmal gewaschen und eine Konjugatlösung bestehend aus Schaf-anti-AFP, an das Peroxidase gekoppelt ist, hinzugegeben. Das Gemisch wird eine Stunde bei Raumtemperatur inkubiert. Anschließend wird erneut ausgesaugt, mit Leitungswasser gewaschen und mit 1 000 µl Substratlösung, die aus ABTS/Wasserstoffperoxid in 100 mM Phosphat-Citrat-Puffer, pH 4,4, besteht, inkubiert. Mit Hilfe von Standardproben mit bekanntem AFP-Gehalt wird eine Eichkurve erstellt. Anhand dieser Eichkurven werden in unbekannten Proben die AFP-Konzentrationen ermittelt.

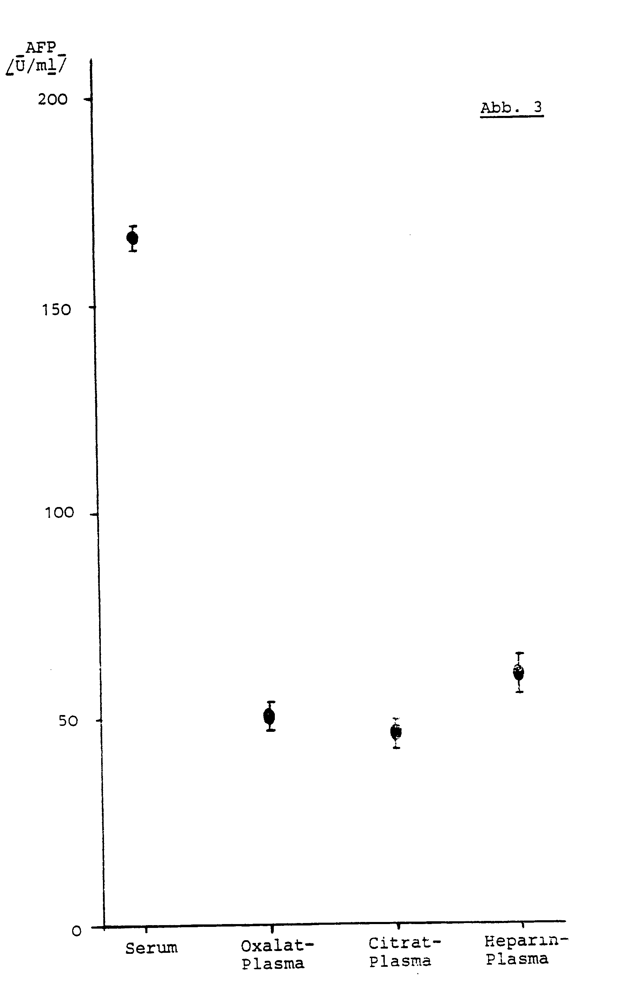

In Abbildung 3 sind die nach obigem Verfahren gefundenen AFP-Werte für Serum, Oxalat-Plasma, Citrat-Plasma und Heparin-Plasma ohne erfindungsgemäßen Zusatz eines Detergens aufgezeigt. Die wiedergegebenen Werte zeigen, daß in Plasmaproben drastisch geringere AFP-Konzentrationen gemessen werden.

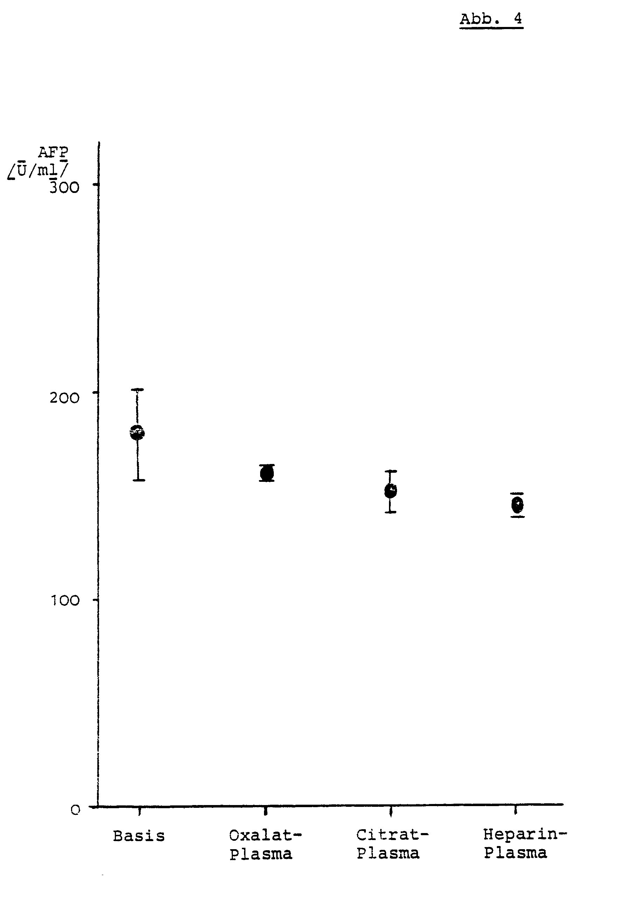

In Abbildung 4 sind die korrespondierenden Werte angegeben, wenn in erfindungsgemäßer Weise bei der Inkubation 0,002 % Tween® 20 zugesetzt wird. Die Abweichungen zwischen Serum und Plasma sind gering. Sie liegen im allgemeinen innerhalb der Fehlergrenze.
Völlig analoge Ergebnisse werden erzielt, wenn bei dem vorstehend beschriebenen Bestimmungsverfahren anstelle von 0,002 % Tween® 20 0,003 % Brij® 35 als Detergens eingesetzt werden.

Beispiel 3

Bestimmung von Thyrotropin (TSH) mit Hilfe eines Sandwich-ELISA-Test

Wie in den Beispielen 1 und 2 beschrieben, werden Kunststoffröhrchen mit monoklonalen Antikörpern der Maus, gerichtet gegen TSH, adsorptiv beschichtet und nach Trocknung wie folgt eingesetzt :
1 000 µl Inkubationslösung, die 100 mM Phosphat-Puffer, pH 6,8 und 1 % Rinderserumalbumin enthält, werden zusammen mit 200 µl Probenlösung aus Serum oder Plasma, die unterschiedliche Mengen TSH enthalten, eine Stunde bei Raumtemperatur inkubiert. Anschließend wird ausgesaugt und gewaschen und mit 1 000 µl Konjugatlösung, enthaltend Schaf-anti-TSH, an das Peroxidase gekoppelt ist, inkubiert. Die Inkubationszeit beträgt eine Stunde bei Raumtemperatur. Danach wird ausgesaugt und gewaschen und wie in Beispiel 1 und 2 angegeben, weitergearbeitet.
Dieses Bestimmungsverfahren wird einmal durchgeführt ohne Zusatz eines Detergens sowie einmal mit Zusatz von 0,005 % Tween® 20. Die für Serum sowie für verschiedene Plasma-Proben gefundenen Werte sind in der folgenden Tabelle gegenüber gestellt.

(Siehe Tabelle Seite 5 f.)

4

Tabelle

Bestimmung von TSH in Serum und verschiedenen Plasma-Proben einmal ohne Zusatz eines Detergens und einmal mit Zusatz von 0,005 % Tween® 20

|  | ohne Zusatz | + 0,005% Tween®20 |
|---|---|---|
| Serum | 13,0 uU/ml | 12,0 uU/ml |
| Oxalat-Plasma | 8,1 uU/ml | 11,2 uU/ml |
| Citrat-Plasma | 9,7 uU/ml | 11,1 uU/ml |
| Heparin-Plasma | 8,6 uU/ml | 10,6 uU/ml |

Auch in diesem Falle zeigt sich, daß ohne Zusatz eines Detergens die Werte für Serum- und Plasma-Proben deutlich voneinander abweichen. Bei erfindungsgemäßem Zusatz eines Detergens stimmen die gemessenen Werte für Serum und Plasma gut überein.

**Patentansprüche**

1. Verfahren zur vergleichbaren Bestimmung eines Partners einer Immunreaktion in Serum- und Plasma-Proben, wobei einer der Reaktionspartner an einen festen Träger adsorbiert ist, dadurch gekennzeichnet, daß dem Inkubationsmilieu der Immunreaktion zwischen dem löslichen Partner bzw. den löslichen Partnern und dem an den festen Träger adsorbierten Bindungspartner ein Detergens zugegeben wird, so daß die Detergens-Konzentration 0,0001 bis 0,01 % (Gew./Vol.) beträgt.

2. Verfahren gemäß Anspruch 1, dadurch gekennzeichnet, daß als Detergens ein nicht-ionisches oder anionisches Detergens zugegeben wird.

3. Verfahren gemäß Anspruch 2, dadurch gekennzeichnet, daß das Detergens ausgewählt wird aus der Gruppe der Polyoxyethylensorbitan-monolaurate, Polyoxyethylen-sorbitan-monooleate, Alkyl-polyether-alkohol-Gemische, Polyoxyethylenlaurylether, Polyoxyethylenoctylphenolether, Polyoxyethylenoxid-alkylether-Addukte, $C_{16}$- oder $C_{18}$-Fettalkohole mit 10 Oxyethylenenheiten und Natriumdodecylsulfat.

4. Verfahren zur Bestimmung eines Akzeptors in einer Serum- oder Plasma-Probe, in dem der Probelösung eine bekannte Menge eines markierten Akzeptors zugegeben wird und der zu bestimmende Akzeptor und der markierte Akzeptor um im Unterschuß vorhandene Bindungsstellen eines Rezeptors, der an einen festen Träger adsorbiert ist, konkurrieren, dadurch gekennzeichnet, daß dem Inkubationsmilieu der Immunreaktion ein Detergens zugesetzt wird, so daß die Detergens-Konzentration 0,0001 bis 0,01 % (Gew./Vol.) beträgt.

5. Verfahren zur Bestimmung eines mindestens bibalenten Akzeptors in einer Serum- oder Plasma-Probe, indem der Akzeptor mit einem markierten Rezeptor und mit einem an einen festen Träger adsorbierten Rezeptor in Kontakt gebracht wird, dadurch gekennzeichnet, daß dem Inkubationsmilieu der Immunreaktion zwischen dem Akzeptor und dem an den festen Träger adsorbierten Rezeptor ein Detergens zugesetzt wird, so daß die Detergenz-Konzentration 0,0001 bis 0,01 % (Gew./Vol.) beträgt.

6. Reagens zur Durchführung einer Immunreaktion zwischen einem an einen festen Träger adsorbierten Reaktionspartner und einem löslichen Partner bzw. löslichen Partnern in einer Serum- oder Plasmaprobe, dadurch gekennzeichnet, daß es zusätzlich zu den für die Immunreaktion erforderlichen Stoffen ein Detergens in einer Konzentration von 0,0001 bis 0,01 % (Gew./Vol.) enthält.

7. Reagens gemäß Anspruch 6, dadurch gekennzeichnet, daß als Detergens ein nicht-ionisches oder anionisches Detergens eingesetzt ist.

**Claims**

1. Process for the comparable determination of a partner of an immune reaction in serum and plasma samples, whereby one of the reaction partners is adsorbed on a solid carrier, characterised in that to the incubation medium of the immune reaction between the soluble partner or the soluble partners and the binding partner adsorbed on the solid carrier there is added a detergent so that the detergent concentration amounts to 0.0001 to 0.01 % (wt./vol.).

2. Process according to claim 1, characterised in that a non-ionic or anionic detergent is added as detergent.

3. Process according to claim 2, characterised in that the detergent is selected from the group of polyoxyethylene-sorbitan monolaurates, polyoxyethylene-sorbitan monoolates, alkyl polyether alcohol

5

mixtures, polyoxyethylene lauryl ethers, polyoxyethylene octylphenolethers, polyoxyethylene oxide alkyl ether adducts, $C_{16}$- or $C_{18}$-fatty alcohols with 10 oxyethylene units and sodium dodecyl sulphate.

4. Process for the determination of an acceptor in a serum or plasma sample in which a known amount of a labelled acceptor is added to the sample solution and the acceptor to be determined and the labelled acceptor compete for binding points present in insufficiency of a receptor which is adsorbed on a solid carrier, characterised in that to the incubation medium of the immune reaction is added a detergent so that the detergent concentration amounts to 0.0001 to 0.01 % (wt./vol.).

5. Process for the determination of an at least bivalent acceptor in a serum or plasma sample, in which the acceptor is brought into contact with a labelled receptor and with a receptor adsorbed on a solid carrier, characterised in that to the incubation medium of the immune reaction between the acceptor and the receptor adsorbed on the solid carrier there is added a detergent so that the detergent concentration amounts to 0.0001 to 0.01 % (wt./vol.).

6. Reagent for the carrying out of an immune reaction between a reaction partner adsorbed on a solid carrier and a soluble partner or soluble partners in a serum or plasma sample, characterised in that, in addition to the materials necessary for the immune reaction, it contains a detergent in a concentration of 0.0001 to 0.01 % (wt./vol.).

7. Reagent according to claim 6, characterised in that, as detergent, there is used a non-ionic or anionic detergent.

## Revendications

1. Procédé pour la détermination comparable d'un partenaire d'une immunoréaction dans des échantillons de sérum et de plasma, un des partenaires réactionnels étant adsorbé sur un support solide, caractérisé en ce qu'il est ajouté au milieu d'incubation de l'immunoréaction entre le ou les partenaires solubles et le partenaire de liaison adsorbé sur un support solide, un détergent dans une proportion telle que la concentration en détergent soit comprise entre 0,0001 et 0,01 % (poids/volume).

2. Procédé selon la revendication 1, caractérisé en ce que comme détergent, on ajoute un détergent non ionique ou anionique.

3. Procédé selon la revendication 2, caractérisé en ce que le détergent est choisi dans le groupe des monolaurate de polyoxyéthylène-sorbitane, monooléate de polyoxyéthylène-sorbitane, mélanges alkylpolyéther-alcool, polyoxyéthylène-lauryléther, polyoxyéthylène-octylphénoléther, adducts polyoxyéthylèneoxyde-alkyléther, alcools gras en $C_{16}$ ou $C_{18}$ comportant 10 motifs d'oxyéthylène et dodécylsulfate de sodium.

4. Procédé pour la détermination d'un accepteur dans un échantillon de sérum ou de plasma, dans lequel on ajoute à la solution d'échantillon une quantité connue d'un accepteur marqué et on met en compétition l'accepteur à déterminer et l'accepteur marqué autour des sites de liaison, en quantité insuffisante, d'un récepteur adsorbé sur un support solide, caractérisé en ce qu'au milieu d'incubation de l'immunoréaction il est ajouté un détergent dans une proportion telle que la concentration en détergent soit comprise entre 0,0001 et 0,01 % (poids/volume).

5. Procédé pour la détermination d'un accepteur au moins bivalent dans un échantillon de sérum ou de plasma, dans lequel l'accepteur est mis en contact avec un récepteur marqué et un récepteur adsorbé sur un support solide, caractérisé en ce qu'au milieu d'incubation réactionnel de l'immunoréaction entre l'accepteur et le récepteur adsorbé sur un support solide, on ajoute un détergent dans une proportion telle que la concentration en détergent soit comprise entre 0,0001 et 0,01 % (poids/volume).

6. Réactif pour la mise en œuvre d'une immunoréaction entre un partenaire réactionnel adsorbé sur un support solide et un ou des partenaires réactionnels solubles dans un échantillon de sérum ou de plasma, caractérisé en ce qu'en plus des matières nécessaires à l'immunoréaction, il contient un détergent en une concentration comprise entre 0,0001 et 0,01 % (poids/volume).

7. Réactif selon la revendication 6, caractérisé en ce que comme détergent, on utilise un détergent non ionique ou anionique.

Y-ACHSE :WIEDERFINDUNG %          Abb. 1

Y-ACHSE :WIEDERFINDUNG %

Abb. 2

Abb. 3

Abb. 4